# EUROPEAN PATENT APPLICATION

(11) **EP 2 394 647 A1**
(43) Date of publication of application: **14.12.2011**
(21) Application number: 11178884.0
(22) Date of filing: 01.11.2007
(51) Int. Cl.: A61K 31/357, A61K 31/433, A61K 31/664, A61P 25/02, A61P 25/04, A61P 43/00, A61K 31/00, A61K 31/437

(54) **Methods of treating neuropathic pain by modulation of glycogenolysis or glycolysis pathways**

(30) Priority: 02.11.2006 US 864095 P
(62) Divisional of application: 07863779.0
(71) Applicant: Aestus Therapeutics, Inc., North Brunswick, NJ 08902 (US)
(72) Inventor: Chiang, Lillian W., Princeton, NJ New Jersey 08540 (US); Honore, Tage, Princeton, NJ New Jersey 08540 (US)
(74) Representative: Jansen, Cornelis Marinus

(57) **Abstract**

Embodiments of the invention relate to the treatment of neuropathic pain in mammals. Embodiments of the invention include methods for treating neuropathic pain as well as methods for preparing medicaments used in the treatment of mammalian pain. Preferably, methods of the invention comprise the modulation of glycogenolysis or glycolysis pathways for the treatment of mammalian pain.

## Description

### Field

Embodiments of the invention relate to the treatment of pain, including neuropathic pain, in mammals.

### Background

### Pain

Pain is the most common symptom for which patients seek medical help, and can be classified as either acute or chronic. Acute pain is precipitated by immediate tissue injury (e.g., a burn or a cut), and is usually self-limited. This form of pain is a natural defense mechanism in response to immediate tissue injury, preventing further use of the injured body part, and withdrawal from the painful stimulus. It is amenable to traditional pain therapeutics, including non-steroidal anti-inflammatory drugs (NSAIDs) and opioids. In contrast, chronic pain is present for an extended period, e.g., for 3 or more months, persisting after an injury has resolved, and can lead to significant changes in a patient's life (e.g., functional ability and quality of life) (Foley, Pain, In: Cecil Textbook of Medicine, pp. 100-107, Bennett and Plum eds., 20th ed., 1996).

Chronic debilitating pain represents a significant medical dilemma. In the United States, about 40 million people suffer from chronic recurrent headaches; 35 million people suffer from persistent back pain; 20 million people suffer from osteoarthritis; 2.1 million people suffer from rheumatoid arthritis; and 5 million people suffer from cancer-related pain (Brower, Nature Biotechnology 2000; 18:387-191). Cancer-related pain results from both inflammation and nerve damage. In addition, analgesics are often associated with debilitating side effects such as nausea, dizziness, constipation, respiratory depression and cognitive dysfunction (Brower, Nature Biotechnology 22000; 18:387-391). Pain can be classified as either "nociceptive" or "neuropathic", as defined below.

"Nociceptive pain" results from activation of pain sensitive nerve fibers, either somatic or visceral. Nociceptive pain is generally a response to direct tissue damage. The term "neuropathic pain" refers to pain that is due to injury or disease of the central or peripheral nervous system. In contrast to the immediate pain caused by tissue injury, neuropathic pain can develop days or months after a traumatic injury. Furthermore, while pain caused by tissue injury is usually limited in duration to the period of tissue repair, neuropathic pain frequently is long lasting or chronic. Moreover, neuropathic pain can occur spontaneously or as a result of stimulation that normally is not painful.

Unfortunately, neuropathic pain is often resistant to available drug therapies; a hallmark of neuropathic pain is its intractability. Typical non-steroidal anti-inflammatory drugs (NSAIDs) such as aspirin, indomethecin, and ibuprofen do not relieve neuropathic pain. The neuropathic pain observed in animal models predictive of human clinical outcome does not respond to NSAIDs. Treatments for neuropathic pain include opioids, anti-epileptics, NMDA antagonists, topical Lidocaine, and tricyclic anti-depressants. Current therapies may have serious side effects such as abuse potential, cognitive changes, sedation, and nausea. Many patients suffering from neuropathic pain have limited tolerance of such side effects.

### Glycogenolysis, Glycolysis and Modulators Thereof

Background information related to glycogen, glycogenolysis, and glycolysis may be found in WO 2006002121 Antiglycolytic compound 2-deoxyglucose for treating seizure and paroxysmal disorders, Seven M. Kriegler, Avtar S. Roopra, Thomas Sutula, Carl E. Stafstrom, January 5, 2006; WO 2005056834, Method for alleviating syndromes and conditions of discomfort of the mammalian intestinal and genito-urinary tract, Slan E. Kligerman, Sarah Finnegan, July 25, 2002; WO 2006069451, Use of resiniferatoxin for producing an agent for treating joint pain or other pain, and method of application, Dominik Meyer, July 6, 2006; WO 2006069452, Use of a Vanilloid Receptor Agonist Together With a Glycosaminoglycan or Proteoglycan for Producing an Agent for Treating Articular Pain, and Application Method, Dominik Meyer, July 6, 2006; WO 2006066419, Mixture of a Vanilloid Receptor Agonist and a Substance Inhibiting Nerve Regeneration, Use Thereof for Producing Painkiller, and Method for Applying the Painkiller, Dominik Meyer, June 29, 2006; US 5045532, Inner esters of Gangliosides with Analgesic Antiinflammatory Activity, Francesco Della Valle, Aurelio Romeo, September 3, 1991.

### CS-917 and related Fructose biphosphatase inhibitors

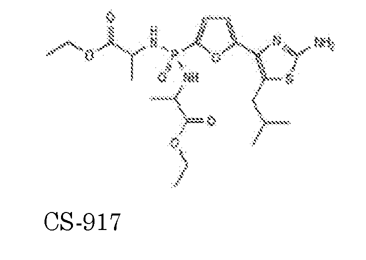

Metabasis (formerly Gensia SICOR which became SICOR), in collaboration with Daiichi Sankyo (formerly Sankyo), is developing the fructose-1,6-bisphosphatase inhibitor CS-917 for the potential treatment of type 2 diabetes. In January 2006, Sankyo began a phase IIb trial; in March 2006, phase III trials were planned for 2007. By May 2006, Daiichi Sankyo was preparing the agent for phase I testing in Japan.

CS-917 and related Fructose Bisphosphatase inhibitors are disclosed in the following publications: Sankyo Co Ltd [Daiichi Sankyo Co Ltd] (Patent Assignee/Owner). Preventive for the onset of diabetes, WO-2004009118.29-JAN-04 (23-JUL-02);

Metabasis Therapeutics Inc (Patent Assignee/Owner), A combination of FBPase inhibitors and antidiabetic agents useful for the treatment of diabetes, WO-00203978.17-JAN-02 (06-JUL-00);

U.S. Patent 6,489,476;

Metabasis Therapeutics Inc. (Patent Assignee/Owner), Novel bisamidate phosphonate prodrugs. WO-00147935.05-JUL-01 (22-DEC-99);

Metabasis Therapeutics Inc (Patent Assignee/Owner) A combination of FBPase inhibitors and insulin sensitizers for the treatment of diabetes. WO-00038666.06-JUL-00 (24-DEC-98); and

Metabasis Therapeutics Inc (Patent Assignee/Owner). Novel heteroaromatic inhibitors of fructose 1,6-bisphosphatase, WO-00014095.16-MAR-00 (09-SEP-98). These references also disclose a genus of fructose-1,6-bisphosphatase inhibitors, including R667, useful for the treatment of type 2 diabetes, with the structural formula: 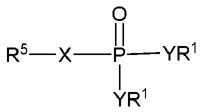 wherein R⁵ is selected from the group consisting of: 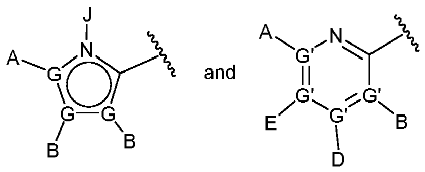 wherein:
each G is independently selected from the group consisting of C, N, O, S, and Se, and wherein no more than one G is O, S, or Se, and at most one G is N;
each G' is independently selected from the group consisting of C and N and wherein no more than two G' groups are N;

A is selected from the group consisting of -H, -NR⁴₂, -CON R⁴₂, - CO₂R³, halo, -S(O)R³, -SO₂R³, alkyl, alkenyl, alkynyl, perhaloalkyl, haloalkyl, aryl, -CH₂OH, -CH₂ N R⁴₂, -CH₂ CN, -CN, -C(S)NH₂, -OR³ -SR³, -N₃, -NHC(S)N R⁴₂, -NHAc, and nothing;
each B and D are independently selected from the group consisting of -H, alkyl, alkenyl, alkynyl, aryl, alicyclic, aralkyl, alkoxyalkyl, -C(O)R¹¹ - C(O)SR³, -SO₂ R¹¹ -S(O)R³, -CN, -NR⁹₂, -OR³, -SR³, perhaloalkyl, halo, -NO₂, and nothing, all except -H, -CN, perhaloalkyl, -NO₂, and halo are substituted or unsubstituted;
E is selected from the group consisting of -H, alkyl, alkenyl, alkynyl, aryl, alicyclic, alkoxyalkyl, -C(O)OR³, -CONR⁴₂, -CN, -NR⁹₂, -NO₂, -OR³, -SR³, perhaloalkyl, halo, and nothing, all except -H, -CN, perhaloalkyl, and halo are substituted or unsubstituted;
J is selected from the group consisting of -H and nothing;
X is a substituted or unsubstituted linking group that links R⁵ to the phosphorus atom via 2-4 atoms, wherein 0-1 atoms are heteroatoms selected from N, O, and S, and the remaining atoms are carbon, except that if X is urea or carbamate there are 2 heteroatoms, measured by the shortest path between R⁵ and the phosphorus atom, and wherein the atom attached to the phosphorus is a carbon atom, and wherein there is no N in the linking group unless it is connected directly to a carbonyl or in the ring of a heterocycle; and wherein X is not a 2 carbon atom -alkyl- or -alkenyl- group; with the proviso that X is not substituted with -COOR², -SO₃R¹, or -PO₃R¹₂ ;
Y is independently selected from the group consisting of -O-, and - NR⁶-;
when Y is -O-, then R¹ attached to -O- is independently selected from the group consisting of -H, alkyl, substituted or unsubstituted aryl, substituted or unsubstituted alicyclic where the cyclic moiety contains a carbonate or thiocarbonate, substituted or unsubstituted -alkylaryl, -C(R²)₂ OC(O)NR²₂, - NR² -C(O)-R³, -C(R²)₂ -OC(O)R³, -C(R²)₂ -O-C(O)OR³, -C(R²)₂ OC(O)SR³, -alkyl-S-C(O)R³, -alkyl-S-S-alkylhydroxy, and -alkyl-S-S-S-alkylhydroxy,
when Y is -NR⁶ -, then R¹ attached to -NR⁶ - is independently selected from the group consisting of -H, -[C(R²)₂]_{q} -COOR³, -C(R⁴)₂ COOR³, - [C(R²)₂]_{q} -C(O)SR³, and -cycloalkylene-COOR³ ;
or when either Y is independently selected from -O- and -NR⁶ -, then together R¹ and R¹ are -alkyl-S-S-alkyl- to form a cyclic group, or together R¹ and R¹ are 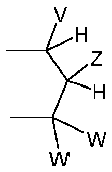 wherein
V, W, and W' are independently selected from the group consisting of -H, alkyl, aralkyl, alicyclic, aryl, substituted aryl, heteroaryl, substituted heteroaryl, 1-alkenyl, and 1-alkynyl; or
together V and Z are connected via an additional 3-5 atoms to form a cyclic group containing 5-7 atoms, wherein 0-1 atoms are heteroatoms and the remaining atoms are carbon, substituted with hydroxy, acyloxy, alkoxycarbonyloxy, or aryloxycarbonyloxy attached to a carbon atom that is three atoms from both Y groups attached to the phosphorus; or
together V and Z are connected via an additional 3-5 atoms to form a cyclic group, wherein 0-1 atoms are heteroatoms and the remaining atoms are carbon, that is fused to an aryl group at the beta and gamma position to the Y attached to the phosphorus;
together V and W are connected via an additional 3 carbon atoms to form a substituted or unsubstituted cyclic group containing 6 carbon atoms and substituted with one substituent selected from the group consisting of hydroxy, acyloxy, alkoxycarbonyloxy, alkylthiocarbonyloxy, and
aryloxycarbonyloxy, attached to one of said carbon atoms that is three atoms from a Y attached to the phosphorus;
together Z and W are connected via an additional 3-5 atoms to form a cyclic group, wherein 0-1 atoms are heteroatoms and the remaining atoms are carbon, and V must be aryl, substituted aryl, heteroaryl, or substituted heteroaryl;
together W and W' are connected via an additional 2-5 atoms to form a cyclic group, wherein 0-2 atoms are heteroatoms and the remaining atoms are carbon, and V must be aryl, substituted aryl, heteroaryl, or substituted heteroaryl;
Z is selected from the group consisting of -CHR² OH, -CHR² OC(O)R³, -CHR² OC(S)R³, -CHR² OC(S)OR³, -CHR² OC(O)SR³, -CHR² OCO₂ R³, -OR², -SR², -CHR² N₃, -CH₂ aryl, -CH(aryl)OH, -CH(CH.dbd.CR²₂)OH, - CH(C.ident.CR²)OH, -R², -NR²₂, -OCOR³, -OCO₂ R³, -SCOR³, -SCO₂ R³, - NHCOR², -NHCO₂ R³, -CH₂ NHaryl, -(CH₂)ₚ -OR², and -(CH₂)ₚ -SR² ;
p is an integer 2 or 3;
q is an integer 1 or 2;
with the provisos that:
a) V, Z, W, W' are not all -H; and
b) when Z is -R², then at least one of V, W, and W' is not -H, alkyl, aralkyl, or alicyclic;
R² is selected from the group consisting of R³ and -H;
R³ is selected from the group consisting of alkyl, aryl, alicyclic, and aralkyl;
each R⁴ is independently selected from the group consisting of -H, and alkyl, or together R⁴ and R⁴ form a cyclic alkyl group;
R⁶ is selected from the group consisting of -H, lower alkyl, acyloxyalkyl, alkoxycarbonyloxyalkyl, and lower acyl;
each R⁹ is independently selected from the group consisting of -H, alkyl, aralkyl, and alicyclic, or together R⁹ and R⁹ form a cyclic alkyl group;
R¹¹ is selected from the group consisting of alkyl, aryl, -NR²₂, and - OR² ; and with the provisos that:
1) when G' is N, then the respective A, B, D, or E is nothing;
2) at least one of A and B, or A, B, D, and E is not selected from the group consisting of -H or nothing;
3) when R⁵ is a six-membered ring, then X is not any 2 atom linker, a substituted or unsubstituted -alkyl-, a substituted or unsubstituted -alkenyl-, a substituted or unsubstituted -alkyloxy-, or a substituted or unsubstituted - alkylthio-;
4) when G is N, then the respective A or B is not halogen or a group directly bonded to G via a heteroatom;
5) R¹ is not unsubstituted C1-C10 alkyl;
6) when X is not an -aryl- group, then R⁵ is not substituted with two or more aryl groups;
and pharmaceutically acceptable salts, hydrates, solvates, prodrugs, and polymorphs thereof.

### Preclinical data

Using rat hepatocytes, isolated rat kidneys and whole animal studies, the drug was shown to inhibit gluconeogenesis.

A combination of CS-917 and troglitazone performed better than CS-917 alone at lowering blood glucose and blood lactate in male ZDF rats.

In overnight fasted cynomolgus monkeys, CS-917 (20 to 60 mg/kg) dose-dependently decreased insulin levels 3 h after administration and plasma glucagons levels were increased at the 60 mg/kg dose.

In fasted goto-kakizaki (GK) rats, a model of non-obese insulin resistant diabetes, CS-917 (5 to 40 mg/kg) dose-dependently decreased plasma glucose levels 3 h after administration and increased plasma glucagons at a dose of 40 mg/kg. CS-917 (20 and 40 mg/kg) also increased hepatic fructose 1,6-bisphosphate and fructose 6-phosphate 4h after administration to fasted GK rats.

When overnight fasted rats were treated with CS-917 (2.5 to 40 mg/kg) 1 h in advance of a liquid meal loading containing 20 kcal/kg, the AUC values of plasma glucose levels were significantly dose dependently decreased at doses over 10 mg/kg.

Female ZDF rats fed a diet of 48% kcal fat for about 30 days were divided into glucose matched groups (n = 8/group) and administered CS-917 as a food mixture at approximate doses of 100 or 300 mg/kg/day for 14 days. CS-917 (100 mg/kg/day) significantly ameliorated hyperglycemia compared with controls (305 +/- 34 versus 166 +/- 24 mg/dl) and polydipsia (29 +/- 12.3 versus 15 +/- 6.2 ml/day). Plasma triglycerides tended to be lowered in CS-917-treated rats, whereas, insulin levels, blood lactate, liver glycogen and liver triglycerides were largely unaffected. Similar reductions in blood glucose without metabolic perturbation were observed with the 300 mg/kg/day dose.

Following a 4 h fast, maximally effective doses of CS-917 (300 mg/kg) and/or glyburide (100 mg/kg) were administered orally to Zucker diabetic fatty (ZDF) rats, followed 90 min later by an oral glucose load of 2 g/kg. Over the 3 h following glucose administration, both drugs improved glucose tolerance; combination treatment was superior to monotherapy and lowered blood glucose below baseline after 2 h post-load. No stimulation of insulin secretion was observed in the vehicle or CS-917 treated group. The positive insulin secretory response to glyburide was identical in the monotherapy and combination groups.

The compound had good oral efficacy in both freely feeding and 6-h fasting ZDF rats and was effective in early and advanced disease states.

CS-917 inhibited glucose production in primary hepatocytes with EC50 values of 300 nM and 1 to 3 microM for human and rat/mouse hepatocytes, respectively. CS-917 effectively lowered glucose in a chronic 21-day study using 10-week old ZDF rats; glucose lowering was irrespective of age and insulin levels of rats; the minimum efficacious dose was 30 mg/kg.

### Clinical data

A total of 39 patients received 50, 100, 200 or 400 mg of CS-917 qd or placebo for 14 days. The drug was rapidly absorbed, extensively metabolized, rapidly cleared and well tolerated with no serious adverse events. Two subjects taking the 400 mg dose had asymptomatic elevated lactic acid levels and the lactic acid AUC was increased in that group compared with placebo. The lactic acid profiles were indifferent from placebo in the other dose groups.

In a single-center, randomized, double-blind study, 96 volunteers were treated with one oral suspension dose of CS-917 (2.5, 5, 10, 25, 50, 100, 200, 400, 600, 800 or 1000 mg) or placebo
after an overnight fast. The drug was well tolerated with nausea seen at 400 mg and vomiting seen at 800 and 1000 mg. Glucose levels were not affected.

In another single-center, randomized, double-blind study, 633 volunteers were treated with oral CS-917 capsules (100, 200, 400 or 800 mg) or placebo for 14 days. The drug was well tolerated with no serious adverse events. Nausea was seen in the 400 and 800 mg groups, and vomiting was seen in the 800 mg group.

In August 2006, Sankyo reported that had completed a 28-day phase IIa trial that confirmed and expanded on these results.

In September 2003, a phase IIa study had been completed. In the randomized, placebo-controlled, double-blind study, patients received CS-917 once daily for 14 days. The results showed that CS-917-treated patients exhibited lower blood glucose levels for the first 6 h after dosing on day 14, compared to glucose levels on the day before the first dose was administered. Moreover, glucose lowering was greater in CS-917-treated patients relative to placebo-treated patients.

Several phase II trials are ongoing.

### NP-12 and related Glycogen Synthase Kinase-3 beta inhibitors

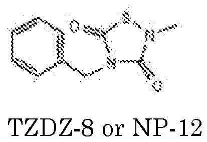

Neuropharma SA is developing NP-12, the lead in a series of oral heterocyclic thiadiazolidinones (TZDs) that inhibit glycogen synthase kinase 3 beta (GSK-3-beta) for the potential treatment of CNS disorders, including Alzheimer's disease.

NP-12 and related GSK-3-beta inhibitors are disclosed in the following references: Consejo Superior De Investigaciones Cientificas (Patent Assignee/Owner), Heterocyclic inhibitors of glycogen synthase kinase GSK-3. WO-00185685.15-NOV-01 (11-MAY-00);

Neuropharma SA [Zeltia SA] (Patent Assignee/Owner) The use of 1, 2, 4-thiadiazolidine-3,5-diones as PPAR activators WO-2006045581.(04-MAY-06);
U.S. Patent 5,532,256;
U.S. Patent 6,872,737;
U.S. Patent Application publication 2003/0195238; and
Neuropharma SA [Zeltia SA] (Patent Assignee/Owner) Use of thiadiazolidine-derived compounds as neurogenic agents WO-2006084934. (17-AUG-06). These references disclose a genus of GSK-3-beta inhibitors useful for treating Alzheimer's, with the structural formula: 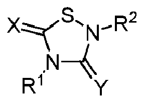 wherein R¹ and R² are independently selected from hydrogen, alkyl, cycloalkyl, haloalkyl, aryl,
-(Z)ₙ-aryl, heteroaryl, -OR³, -C(O)R³, -C(O)OR³, -(Z)ₙ-C(0)0 R³, and - S(O)t-; where
X and Y are independently selected from S and O, and at least one of X and Y is O;
n is 0, 1 or 2;
t is 0, 1, or 2;
R³ and R⁴ are independently selected from hydrogen, alkyl, aryl, and heterocyclic; and
Z is independently selected from -C(R³)(R⁴)-, -C(O)-, -O-, -C(=NR3)-, - S(O)ₜ-, and -N(R3);
and pharmaceutically acceptable salts, hydrates, solvates, prodrugs, and polymorphs thereof.

### Preclinical data

Double transgenic Tet/GSK-3-beta mice were treated orally with either NP-01139 (50 to 100 mg/kg/day) or NP-12 (100 to 200 mg/kg/day). After 3 weeks of treatment, their spatial learning capabilities were studied in the Morris water maze and biochemical and immunohistochemical analysis of brain samples was performed. Prolonged oral treatment of Tet/GSK-3-beta mice with both compounds induced a dose-dependent significant decrease in tau phosphorylation in the hippocampus, while not showing any apparent clinical signs.

### Clinical data

A two-stage phase I trial dose study and a 7 day repeated-dose study has been initiated..

### PSN-357 and related Glycogen Phosphorylase inhibitors

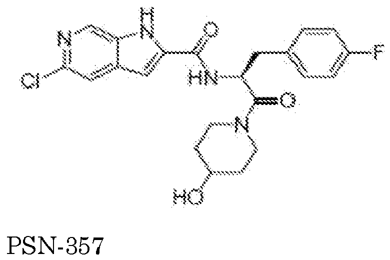

(OSI) Prosidion (formerly Prosidion) is developing PSN-357 and acid addition salts of PSN-357, an oral glycogen phosphorylase inhibitor, for the potential treatment of type 2 diabetes. Conjugate acids include HF, HCl, HBr, HI, H₂SO₄, RCO₂H, HClO₄, H₃PO₄, and RSO₃H where R is alkyl, substituted alkyl, aryl, substituted aryl, heteroaryl, or substituted heteroaryl.

PSN-357 and related pyrrolopyridine-2-carboxylic acid amide inhibitors of glycogen phosphorylase disclosed in the following references:
(OSI) Prosidion [OSI Pharmaceuticals Inc] (Patent Assignee/Owner), Pyrrolopyridine-2-carboxylic acid amide derivative useful as inhibitor of glycogen phosphorylase, WO-2006059165. (08-JUN-06) and (OSI) Prosidion [OSI Pharmaceuticals Inc] (Patent Assignee/Owner), Pyrrolopyridine-2-carboxylic acid amide inhibitors of glycogen phosphorylase, WO-2004104001. (02-DEC-04). These references disclose a genus of glycogen phosphorylase inhibitors useful for treating type 2 diabetes, of the structural formula: 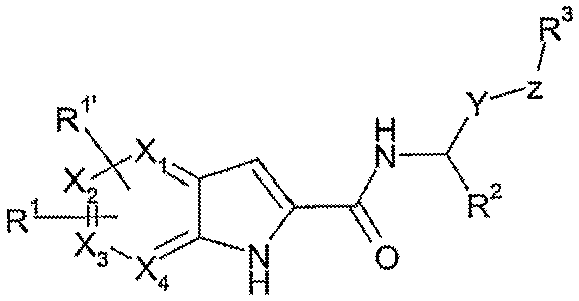
or a stereoisomer, or a pharmaceutically acceptable salt thereof, wherein:
one of X₁, X₂, X₃ and X₄ must be N and the others must be C;
R¹ and R^{1'} are each independently, halogen, hydroxy, cyano, C₀₋₄alkyl, C₁₋₄alkoxy, fluoromethyl, difluoromethyl, trifluoromethyl, ethenyl, or ethynyl;
R² is CO-4alkyl, COOR6, COR6, C₁₋₄alkoxyC₁₋₄alkyl-, hydroxyC₁₋₄alkyl-, cycloalkylC₀₋₄alkyl-, arylC₀₋₄alkyl-, hetarylC₀₋₄alkyl-, wherein any of the aryl or hetaryl rings are optionally substituted with 1-2 independent halogen, cyano, C₁₋₄alkyl, C₁₋₄alkoxy, -N(C₀₋₄alkyl)(C₀₋₄alkyl),-SO₂C₁₋₄alkyl,-SO₂N(C₀₋₄alkyl)(C₀₋₄alkyl), hydroxy, fluoromethyl, difluoromethyl, or trifluoromethyl substituents;
Y is C₀₋₂alkyl or-CH(OH)- ;
Z is CH₂, -C(O)-, -O-, >N(C₀₋₄alkyl), >N(C₃₋₆cycloalkyl), or absent; but when Y is -CH(OH) -, Z or R³ must be bonded to Y through a carbon-carbon bond;
R³ is hydrogen, -COOC₀₋₄alkyl, C₁₋₄alkoxy, C₁₋₄alkyl, arylC₁₋₄alkylthio-, -C₀₋₄alkylaryl, -C₀₋₄alkylhetaryl, -C₀₋₄alkylcycloalkyl or -C₀₋₄alkylheterocyclyl, wherein any of the rings is optionally substituted with 1-3 independent halogen, cyano, C₁₋₄alkyl, fluoromethyl, difluoromethyl, trifluoromethyl, -C₀₋₄alkylNHC(O)O(C₁₋₄alkyl), -C₀₋₄alkylNR⁷R⁸, -C(O)R⁹, C_{1- 4}alkoxyC₀₋₄alkyl-, -COOC₀₋₄alkyl, -C_{0- 4}alkylNHC(O)R⁹, -C₀₋₄alkylC(O)N(R¹⁰)₂, - C₁₋₄alkoxyC₁₋₄alkoxy, hydroxyC₀₋₄alkyl-, -NHSO₂R¹⁰, -SO₂(C₁₋₄alkyl), - SO₂NR¹¹R¹², 5- to 6-membered heterocyclyl, phenylC₀₋₂alkoxy, or phenylC₀₋₂alkyl substituents, wherein phenyl is optionally substituted with 1-2 independent halogen, cyano, C₁₋₄alkyl, C₁₋₄alkoxy, -N(C₀₋₄alkyl)(C₀₋₄alkyl), S0₂C₁₋₄alkyl, -SO₂N(C₀₋₄alkyl)(C₀₋₄alkyl), hydroxy, fluoromethyl, difluoromethyl or trifluoromethyl substituents, or two bonds on a ring carbon of the heterocyclyl group optionally can form an oxo (=O) substituent;
or R³ is-NR⁴(-C₀₋₄alkylR⁵) ;
R⁴ is C₀₋₃alkyl, -C₂₋₃alkyl-NR⁷R⁸, C₃₋₆cycloalkyl optionally substituted by hydroxyC₀₋₄alkyl-further optionally substituted by hydroxy, C₁₋₂alkoxyC₂₋₄alkyl-, or C₁₋₂alkyl-S(O)ₙ-C₂₋₃alkyl-;
n is 0, 1, or 2 ;
R⁵ is hydrogen, hydroxyC₂₋₃alkyl-, C₁₋₂alkoxyC₀₋₄alkyl-, or aryl, hetaryl, or heterocyclyl;
   wherein a heterocyclic nitrogen-containing R⁵ ring optionally is mono-substituted on the ring nitrogen with C₁₋₄alkyl, benzyl, benzoyl, C₁₋₄alkyl-C(O)-, -SO₂C₁₋₄alkyl, -SO₂N(C₀₋₄alkyl)(C₀₋₄alkyl), C₁₋₄alkoxycarbonyl or aryl(C₁₋₄alkoxy)carbonyl; and wherein the R⁵ rings are optionally mono-substituted on a ring carbon with halogen, cyano, C₁₋₄alkyl-C(O) -, C₁₋₄alkyl-SO₂-, C₁₋₄alkyl, C₁₋₄alkoxy, hydroxy, -N(C₀₋₄alkyl)(C₀₋₄alkyl), hydroxyC₀₋₄alkyl-, or C₀₋₄alkylcarbamoyl-, provided that no quaternised nitrogen is included; or two bonds on a ring carbon of the heterocyclyl group optionally can form an oxo (=O) substituent;
   R⁶ is C₁₋₄alkyl, aryl, or hetaryl;
   R⁷ and R⁸ are independently C₀₋₄alkyl, C₃₋₆cycloalkyl, or CO(C₁₋₄alkyl) ;
   R⁹ is C₁₋₄alkyl or C₃₋₆cycloalkyl ;
   R¹⁰ is C₀₋₄alkyl or C₃₋₆cycloalkyl ;
   R¹¹ and R¹² are independently C₀₋₄alkyl or together with the nitrogen to which they are attached may form a 4-to 6-membered heterocycle; and
wherein there are no nitrogen-oxygen, nitrogen-nitrogen or nitrogen-halogen bonds in linking the three components -Y-Z-R³ to each other.

### Preclinical data

PSN-357 reduced glucagon-induced glycogenolysis in both human and rat hepatocytes with EC₅₀ values of 1.1 and 1.9 microM, respectively.

The compound had a t_{1/2} of 4 h in rats and displayed dose-dependent inhibition of glucagon- (10 microg/kg iv) stimulated elevation of blood glucose when administered to healthy conscious rats with a minimum effective dose of 3 mg/kg.

In ob/ob mice, PSN-357 (30 mg/kg po) reduced blood glucose levels at 90 to 210 minutes post administration with a 66% increase in liver glycogen after 5 h. More sustained glucose lowering was achieved in a similar study in db/db mice with a 35% reduction maintained after 5 h accompanied by an increase in liver glycogen; heart and skeletal muscle glycogen remained unaffected.

In a 9-day study in freely fed db/db mice, administration of PSN-357 (60 mg/kg po qd) prevented hyperglycemia for the duration of the study with a 57% increase in liver glycogen but no changes in muscle glycogen, plasma insulin or alanine aminotransferase.

### Clinical data.

The single-center, open-label trial in 112 healthy volunteers employed a combined single and multiple dose-escalation design, results had shown that the drug was safe and well tolerated.

In February 2006, OSI began a 6-month, placebo-controlled, dose-escalation phase IIa study that would test daily doses of the drug in 30 patients for 14 days.

References cited in the preceding discussion and all other references cited in this application are hereby incorporated herein by reference in their entirety.

### Summary

Pain in mammals is treated by the administration of a therapeutically effective amount of a modulator of glycogenolysis or glycolysis, wherein the modulator is a compound of Formula I, Formula II or Formula III or a pharmaceutically acceptable salt, solvate, ester or hydrate thereof. 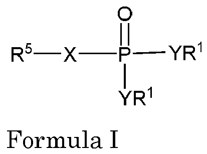 wherein R⁵ is selected from the group consisting of: 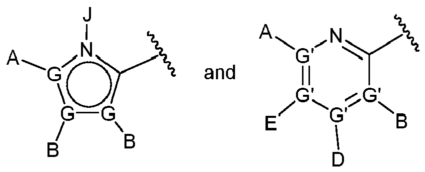 wherein:
each G is independently selected from the group consisting of C, N, O, S, and Se, and wherein no more than one G is O, S, or Se, and at most one G is N;
each G' is independently selected from the group consisting of C and N and wherein no more than two G' groups are N;
A is selected from the group consisting of -H, -NR⁴₂, -CON R⁴₂, - CO₂R³, halo, -S(O)R³, -SO₂R³, alkyl, alkenyl, alkynyl, perhaloalkyl, haloalkyl, aryl, -CH₂OH, -CH₂ N R⁴₂, -CH₂ CN, -CN, -C(S)NH₂, -OR³ -SR³, -N₃, -NHC(S)N R⁴₂, -NHAc, and nothing;
each B and D are independently selected from the group consisting of -H, alkyl, alkenyl, alkynyl, aryl, alicyclic, aralkyl, alkoxyalkyl, -C(O)R¹¹ - C(O)SR³, -SO₂ R¹¹ -S(O)R³, -CN, -NR⁹₂, -OR³, -SR³, perhaloalkyl, halo, -NO₂, and nothing, all except -H, -CN, perhaloalkyl, -NO₂, and halo are substituted or unsubstituted;
E is selected from the group consisting of -H, alkyl, alkenyl, alkynyl, aryl, alicyclic, alkoxyalkyl, -C(O)OR³, -CONR⁴₂, -CN, -NR⁹₂, -NO₂, -OR³, -SR³, perhaloalkyl, halo, and nothing, all except -H, -CN, perhaloalkyl, and halo are substituted or unsubstituted;
J is selected from the group consisting of -H and nothing;
X is a substituted or unsubstituted linking group that links R⁵ to the phosphorus atom via 2-4 atoms, wherein 0-1 atoms are heteroatoms selected from N, O, and S, and the remaining atoms are carbon, except that if X is urea or carbamate there are 2 heteroatoms, measured by the shortest path between R⁵ and the phosphorus atom, and wherein the atom attached to the phosphorus is a carbon atom, and wherein there is no N in the linking group unless it is connected directly to a carbonyl or in the ring of a heterocycle; and wherein X is not a 2 carbon atom -alkyl- or -alkenyl- group; with the proviso that X is not substituted with -COOR², -SO₃ R¹, or -PO₃ R¹₂ ;
Y is independently selected from the group consisting of -O-, and - NR⁶-;
when Y is -O-, then R¹ attached to -O- is independently selected from the group consisting of -H, alkyl, substituted or unsubstituted aryl, substituted or unsubstituted alicyclic where the cyclic moiety contains a carbonate or thiocarbonate, substituted or unsubstituted -alkylaryl, -C(R²)₂ OC(O)NR²₂, - NR² -C(O)-R³, -C(R²)₂ -OC(O)R³, -C(R²)₂ -O-C(O)OR³, -C(R²)₂ OC(O)SR³, -alkyl-S-C(O)R³, -alkyl-S-S-alkylhydroxy, and -alkyl-S-S-S-alkylhydroxy,
when Y is -NR⁶ -, then R¹ attached to -NR⁶ - is independently selected from the group consisting of-H, -[C(R²)₂ ]q -COOR³, -C(R⁴)₂ COOR³, - [C(R²)₂ ]q -C(O)SR³, and -cycloalkylene-COOR³ ;
or when either Y is independently selected from -O- and -NR⁶ -, then together R¹ and R¹ are -alkyl-S-S-alkyl- to form a cyclic group, or together R¹ and R¹ are 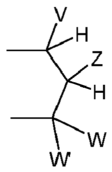 wherein
V, W, and W' are independently selected from the group consisting of -H, alkyl, aralkyl, alicyclic, aryl, substituted aryl, heteroaryl, substituted heteroaryl, 1-alkenyl, and 1-alkynyl; or
together V and Z are connected via an additional 3-5 atoms to form a cyclic group containing 5-7 atoms, wherein 0-1 atoms are heteroatoms and the remaining atoms are carbon, substituted with hydroxy, acyloxy, alkoxycarbonyloxy, or aryloxycarbonyloxy attached to a carbon atom that is three atoms from both Y groups attached to the phosphorus; or
together V and Z are connected via an additional 3-5 atoms to form a cyclic group, wherein 0-1 atoms are heteroatoms and the remaining atoms are carbon, that is fused to an aryl group at the beta and gamma position to the Y attached to the phosphorus;
together V and W are connected via an additional 3 carbon atoms to form a substituted or unsubstituted cyclic group containing 6 carbon atoms and substituted with one substituent selected from the group consisting of hydroxy, acyloxy, alkoxycarbonyloxy, alkylthiocarbonyloxy, and aryloxycarbonyloxy, attached to one of said carbon atoms that is three atoms from a Y attached to the phosphorus;
together Z and W are connected via an additional 3-5 atoms to form a cyclic group, wherein 0-1 atoms are heteroatoms and the remaining atoms are carbon, and V must be aryl, substituted aryl, heteroaryl, or substituted heteroaryl;
together W and W' are connected via an additional 2-5 atoms to form a cyclic group, wherein 0-2 atoms are heteroatoms and the remaining atoms are carbon, and V must be aryl, substituted aryl, heteroaryl, or substituted heteroaryl;
Z is selected from the group consisting of -CHR² OH, -CHR² OC(O)R³, -CHR² OC(S)R³, -CHR² OC(S)OR³, -CHR² OC(O)SR³, -CHR² OCO₂ R³, -OR², -SR², -CHR² N₃, -CH₂ aryl, -CH(aryl)OH, -CH(CH.dbd.CR²₂)OH, - CH(C.ident.CR²)OH, -R², -NR²₂, -OCOR³, -OCO₂ R³, -SCOR³, -SCO₂ R³, - NHCOR², -NHCO₂ R³, -CH₂ NHaryl, -(CH₂)ₚ -OR², and -(CH₂)ₚ -SR² ;
p is an integer 2 or 3;
q is an integer 1 or 2;
with the provisos that:
a) V, Z, W, W' are not all -H; and
b) when Z is -R², then at least one of V, W, and W' is not -H, alkyl, aralkyl, or alicyclic;
R² is selected from the group consisting of R³ and -H;
R³ is selected from the group consisting of alkyl, aryl, alicyclic, and aralkyl;
each R⁴ is independently selected from the group consisting of -H, and alkyl, or together R⁴ and R⁴ form a cyclic alkyl group;
R⁶ is selected from the group consisting of -H, lower alkyl, acyloxyalkyl, alkoxycarbonyloxyalkyl, and lower acyl;
each R⁹ is independently selected from the group consisting of -H, alkyl, aralkyl, and alicyclic, or together R⁹ and R⁹ form a cyclic alkyl group;
R¹¹ is selected from the group consisting of alkyl, aryl, -NR²₂, and - OR² ; and with the provisos that:
1) when G' is N, then the respective A, B, D, or E is nothing;
2) at least one of A and B, or A, B, D, and E is not selected from the group consisting of -H or nothing;
3) when R⁵ is a six-membered ring, then X is not any 2 atom linker, a substituted or unsubstituted -alkyl-, a substituted or unsubstituted -alkenyl-, a substituted or unsubstituted -alkyloxy-, or a substituted or unsubstituted - alkylthio-;
4) when G is N, then the respective A or B is not halogen or a group directly bonded to G via a heteroatom;
5) R¹ is not unsubstituted C1-C10 alkyl;
6) when X is not an -aryl- group, then R⁵ is not substituted with two or more aryl groups. 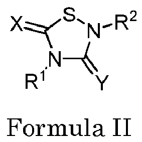
wherein R¹ and R² are independently selected from hydrogen, alkyl, cycloalkyl, haloalkyl, aryl,
-(Z)ₙ-aryl, heteroaryl, -OR³, -C(O)R³, -C(O)OR³, -(Z)ₙ-C(O)O R³, and - S(O)t-; where
X and Y are independently selected from S and O, and at least one of X and Y is O;
n is 0, 1 or 2;
t is 0, 1, or 2;
R³ and R⁴ are independently selected from hydrogen, alkyl, aryl, and heterocyclic; and
Z is independently selected from -C(R³)(R⁴)-, -C(O)-, -O-, -C(=N R³)-, - S(O)ₜ-, and -N(R3) 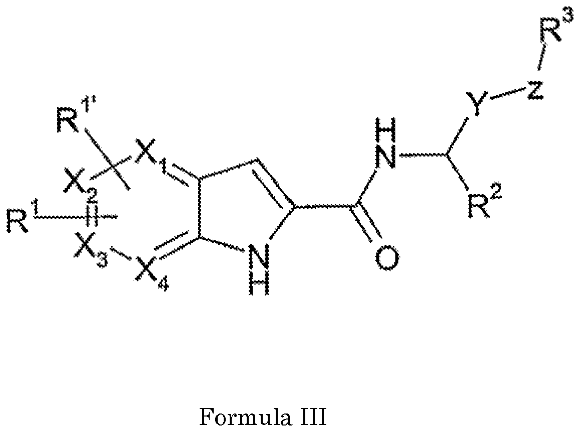 wherein:
one of X₁, X₂, X₃ and X₄ must be N and the others must be C;
R¹ and R^{1'} are each independently, halogen, hydroxy, cyano, C₀₋₄alkyl, C₁₋₄alkoxy, fluoromethyl, difluoromethyl, trifluoromethyl, ethenyl, or ethynyl;
R² is C0-4alkyl, COOR6, COR6, C₁₋₄alkoxyC₁₋₄alkyl-, hydroxyC₁₋₄alkyl-, cycloalkylC₀₋₄alkyl-, arylC₀₋₄alkyl-, hetarylC₀₋₄alkyl-, wherein any of the aryl or hetaryl rings are optionally substituted with 1-2 independent halogen, cyano, C₁₋₄alkyl, C₁₋₄alkoxy, -N(C₀₋₄alkyl)(C₀₋₄alkyl),-SO₂C₁₋₄alkyl,-SO₂N(C₀₋₄alkyl)(C₀₋₄alkyl), hydroxy, fluoromethyl, difluoromethyl, or trifluoromethyl substituents;
Y is C₀₋₂alkyl or-CH(OH)- ;
Z is CH₂, -C(O)-, -O-, >N(C₀₋₄alkyl), >N(C₃₋₆cycloalkyl), or absent; but when Y is -CH(OH) -, Z or R³ must be bonded to Y through a carbon-carbon bond;
R³ is hydrogen, -COOC₀₋₄alkyl, C₁₋₄alkoxy, C₁₋₄alkyl, arylC₁₋₄alkylthio-, -C₀₋₄alkylaryl, -C₀₋₄alkylhetaryl, -C₀₋₄alkylcycloalkyl or -C₀₋₄alkylheterocyclyl, wherein any of the rings is optionally substituted with 1-3 independent halogen, cyano, C₁₋₄alkyl, fluoromethyl, difluoromethyl, trifluoromethyl, -C₀₋₄alkylNHC(O)O(C₁₋₄alkyl), -C₀₋₄alkylNR⁷R⁸, -C(O)R⁹, C₁₋₄alkoxyC₀₋₄alkyl-, -COOC₀₋₄alkyl, -Co- ₄alkylNHC(O)R⁹, -C₀₋₄alkylC(O)N(R¹⁰)₂, - C₁₋₄alkoxyC₁₋₄alkoxy, hydroxyC₀₋₄alkyl-, -NHSO₂R¹⁰, -SO₂(C₁₋₄alkyl), - SO₂NR¹¹R¹², 5- to 6-membered heterocyclyl, phenylC₀₋₂alkoxy, or phenylC₀₋₂alkyl substituents, wherein phenyl is optionally substituted with 1-2 independent halogen, cyano, C₁₋₄alkyl, C₁₋₄alkoxy, -N(C₀₋₄alkyl)(C₀₋₄alkyl), - S0₂C₁₋₄alkyl, -S0₂N(C₀₋₄alkyl)(C₀₋₄alkyl), hydroxy, fluoromethyl, difluoromethyl or trifluoromethyl substituents, or two bonds on a ring carbon of the heterocyclyl group optionally can form an oxo (=O) substituent;
or R³ is-NR⁴(-C₀₋₄alkylR⁵) ;
R⁴ is C₀₋₃alkyl, -C₂₋₃alkyl-NR⁷R⁸, C₃₋₆cycloalkyl optionally substituted by hydroxyC₀₋₄alkyl-further optionally substituted by hydroxy, C₁₋₂alkoxyC₂₋₄alkyl-, or C₁₋₂alkyl-S(O)ₙ-C₂₋₃alkyl-;
n is 0, 1, or 2 ;
R⁵ is hydrogen, hydroxyC₂₋₃alkyl-, C₁₋₂alkoxyC₀₋₄alkyl-, or aryl, hetaryl, or heterocyclyl;
wherein a heterocyclic nitrogen-containing R⁵ ring optionally is mono-substituted on the ring nitrogen with C₁₋₄alkyl, benzyl, benzoyl, C₁₋₄alkyl-C(O)-, -SO₂C₁₋₄alkyl, -SO₂N(C₀₋₄alkyl)(C₀₋₄alkyl), C₁₋₄alkoxycarbonyl or aryl(C₁₋₄alkoxy)carbonyl; and wherein the R⁵ rings are optionally mono-substituted on a ring carbon with halogen, cyano, C₁₋₄alkyl-C(O) -, C₁₋₄alkyl-SO₂-, C₁₋₄alkyl, C₁₋₄alkoxy, hydroxy, -N(C₀₋₄alkyl)(C₀₋₄alkyl), hydroxyC₀₋₄alkyl-, or C₀₋₄alkylcarbamoyl-, provided that no quaternised nitrogen is included; or two bonds on a ring carbon of the heterocyclyl group optionally can form an oxo (=O) substituent;
R⁶ is C₁₋₄alkyl, aryl, or hetaryl;
R⁷ and R⁸ are independently C₀₋₄alkyl, C₃₋₆cycloalkyl, or CO(C₁₋₄alkyl) ;
R⁹ is C₁₋₄alkyl or C₃₋₆-scycloalkyl ;
R¹⁰ is C₀₋₄alkyl or C₃₋₆cycloalkyl ;
R¹¹ and R¹² are independently C₀₋₄alkyl or together with the nitrogen to which they are attached may form a 4-to 6-membered heterocycle; and
wherein there are no nitrogen-oxygen, nitrogen-nitrogen or nitrogen-halogen bonds in linking the three components -Y-Z-R³ to each other.

An embodiment of the invention is a composition for the treatment of neuropathic pain comprising at least one compound selected from the group consisting of a fructose-1,6-bisphosphatase inhibitor of Formula I, a glycogen synthase kinase-3 beta inhibitor of Formula II, or a glycogen phosphorylase inhibitor of Formula III or a salt, ester, hydrate, solvate, prodrug or polymorph thereof, incorporated in a pharmaceutically acceptable adjuvant, excipient, diluent or carrier composition.

An embodiment of the invention is a method of treating neuropathic pain in a mammal in need of such treatment, comprising administering a therapeutically effective amount of a compound selected from the group consisting of a fructose-1,6-bisphosphatase inhibitor of Formula I, a glycogen synthase kinase-3 beta inhibitor of Formula II, or a glycogen phosphorylase inhibitor of Formula III or a salt, ester, hydrate, solvate, prodrug or polymorph thereof.

An embodiment of the invention is a method of treating neuropathic pain in a mammal in need of such treatment comprising administering a therapeutically effective amount of a compound selected from the group consisting of CS-917, NP-12, and PSN-357 and salts, esters, hydrates, solvates, prodrugs, and polymorphs thereof.

Another embodiment of the invention comprises compositions used for treating neuropathic pain comprising at least one compound selected from the group consisting of CS-917, NP-12, and PSN-357 and salts, esters, hydrates, solvates, prodrugs, and polymorphs thereof, incorporated in a pharmaceutically acceptable adjuvant, excipient, diluent, or carrier composition.

In any of the methods for treating pain, the type of pain can be any type of pain, including neuropathic pain, nociceptive pain, chronic pain, pain associated with cancer, and pain associated with rheumatic disease. In one embodiment of the invention, the pain is neuropathic pain.

Compounds of the invention may be administered in a variety of forms. These include, for example, solid, semi-solid and liquid dosage forms, such as tablets, pills, powders, liquid solutions or suspensions, liposomes, nasal/aerosolized dosage forms, implants, injectable and infusible solutions. Compounds may be used as their salts. Typical salts include lithium, sodium, potassium, aluminum, magnesium, calcium, zinc, manganese, ammonium salts and the like and mixtures thereof. In addition, salts may include salts formed with acids such as organic acids or inorganic acids. Typical acids used to form salts may include HF, HCl, HBr, HI, sulfuric, perchloric, phosphoric, acetic, formic, propionic, butyric, pentanoic, benzoic, and the like.

The active compounds can be administered alone or in combination with pharmaceutically acceptable carriers or diluents by any of several routes. More particularly, the active compounds can be administered in a wide variety of different dosage forms, e.g., they may be combined with various pharmaceutically acceptable inert carriers in the form of tablets, capsules, transdermal patches, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents. In addition, oral pharmaceutical compositions can be suitably sweetened and/or flavored. In general, the active compounds are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc can be used for tableting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration the active ingredient may be combined with various sweetening or flavoring agents, coloring matter and, if so desired, emulsifying and/or suspending agents, together with such diluents as water, ethanol, propylene glycol, glycerin and various combinations thereof.

For parenteral administration, a solution of an active compound in either sesame or peanut oil or in aqueous propylene glycol can be employed. The aqueous solutions should be suitably buffered, if necessary, and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

It is also possible to administer the active compounds topically and this can be done by way of creams, a patch, jells, gels, pastes, ointments and the like, in accordance with standard pharmaceutical practice.

The dosage of a specific active compound of the invention depends upon many factors that are well known to those skilled in the art, for example: the particular compound; the condition being treated; the age, weight, and clinical condition of the recipient patient; and the experience and judgment of the clinician or practitioner administering the therapy. An effective amount of the compound is that which provides either subjective relief of symptoms or an objectively identifiable improvement as noted by the clinician or other qualified observer. The dosing range varies with the compound used, the route of administration and the potency of the particular compound.

### Detailed Description

Embodiments of the invention provide methods for treating pain, particularly neuropathic pain.

Embodiments of the invention provide methods for treating pain, particularly neuropathic pain, by modulating glycogenolysis or glycolysis by administering to a subject in need of pain treatment a therapeutically effective amount of a compound that inhibits fructose-1,6-bisphosphatase, glycogen synthase kinase-3 beta, or glycogen phosphorylase. In one embodiment of the invention,

According to embodiments of the invention, a therapeutically effective amount of a compound that inhibits glycogenolysis or glycolysis is administered to a subject to treat pain. A compound useful in carrying out a therapeutic method embodiments of the invention is advantageously formulated in a pharmaceutical composition in combination with a pharmaceutically acceptable carrier. The amount of compound in the pharmaceutical composition depends on the desired dosage and route of administration. In one embodiment, suitable dose ranges of the active ingredient are from about 0.01 mg/kg to about 1500 mg/kg of body weight taken at necessary intervals (e.g., daily, every 12 hours, etc.). In another embodiment, a suitable dosage range of the active ingredient is from about 0.2 mg/kg to about 150 mg/kg of body weight taken at necessary intervals. In another embodiment, a suitable dosage range of the active ingredient is from about 1 mg/kg to about 15 mg/kg of body weight taken at necessary intervals.

In one embodiment, the dosage and administration are such that the glycogenolysis or glycolysis, pathway is only partially modulated so as to avoid any unacceptably deleterious effects.

A therapeutically effective compound can be provided to the subject in a standard formulation that includes one or more pharmaceutically acceptable additives, such as excipients, lubricants, diluents, flavorants, colorants, buffers, and disintegrants. The formulation may be produced in unit dosage from for administration by oral, parenteral, transmucosal, intranasal, rectal, vaginal, or transdermal routes. Parenteral routes include intravenous, intra-arterial, intramuscular, intradermal subcutaneous, intraperitoneal, intraventricular, intrathecal, and intracranial administration.

The pharmaceutical composition can be added to a retained physiological fluid such as blood or synovial fluid. In one embodiment for CNS administration, a variety of techniques are available for promoting transfer of the therapeutic agent across the blood brain barrier, or to gain entry into an appropriate cell, including disruption by surgery or injection, co-administration of a drug that transiently opens adhesion contacts between CNS vasculature endothelial cells, and co-administration of a substance that facilitates translocation through such cells. In another embodiment, for example, to target the peripheral nervous system (PNS), the pharmaceutical composition has a restricted ability to cross the blood brain barrier and can be administered using techniques known in the art.

In another embodiment, the glycogenolysis or glycolysis -modulating compound is delivered in a vesicle, particularly a liposome. In one embodiment, the glycogenolysis or glycolysis-modulating compound is delivered topically (e.g., in a cream) to the site of pain (or related disorder) to avoid the systemic effects of modulating glycogenolysis or glycolysis in non-target cells or tissues.

In another embodiment, the therapeutic agent is delivered in a controlled release manner. For example, a therapeutic agent can be administered using intravenous infusion with a continuous pump, or in a polymer matrix such as poly-lactic/glutamic acid (PLGA), or in a pellet containing a mixture of cholesterol and the active ingredient, or by subcutaneous implantation, or by transdermal patch.

Three independent microarray studies, Chiang et al (patent publication WO 2005/014849 A2), Valder et al (Neurochem, 2003. 87:560), and Wang et al (Neuroscience, 2002. 114:529), were reported for the rat spinal nerve ligation (SNL) model of neuropathic pain. Each of the three groups performed gene expression analysis using the Affymetrix platform on RNA extracted from dorsal root ganglia tissue isolated from rats subjected to SNL. The information on genes reported as regulated by significance criteria specific to each study were combined using Aestus Therapeutics Inc (ATx) proprietary methods. By combining the three datasets for analysis and applying ATx multidimensional analysis, two pathways heretofore never reported as important for neuropathic pain were identified. These pathways are glycogen metabolism or catabolism including glycogenolysis and glycolysis.

Metabolic coupling between glia/astrocytes and neurons is an essential process because neurons can not metabolize glucose directly (for review see Trends Neurosci. 26:536; J. Neurosci. 16:877). Glucose is transported by astrocytes via transporters on end-foot processes to blood vessels. Within astrocytes, glucose is used for synthesis of glycogen for storage. Glycolysis leads to production of lactate. Lactate is transported to neurons for oxidative metabolism. Glycogen turnover in astrocytes increases with neuronal activity to provide the extra energy required. The astrocyte-neuron lactate shuttle is critical for metabolic support of action potentials. Therefore, a decrease in glycolysis would ameliorate a pain state caused by ectopic firing. Modulating targets to decrease glycolysis include mechanisms of action previously developed for the treatment of metabolic disorders such as Type II diabetes. These include inhibition of the glycogen synthase kinase-3 beta (GSK-3 β) and the glycogen phosphorylase.

GSK-3 β phosphorylates glycogen synthase, the key regulator of glycogenolysis (Martinez Gil, A., et al., Heterocyclic inhibitors of glycogen synthase kinase GSK-3, in World Intellectual Property Organization. 2001, Consejo Superior De Investigaciones Cientificas. p. 1-31; Martinez Gil, A., et al., Use of thiadiazolidine-derived compounds as neurogenic agents, in World Intellectual Property Organization. 2006, Neuropharma, S.A. p. 1-47). As a result, the metabolic balance is shifted towards glycogen and substrate availability for conversion to lactate is decreased. Resulting decreased ectopic firing of nociceptive neurons would ameliorate a neuropathic pain state. NP-12 and the other compounds represented by Formula II inhibit GSK-3 β.

PSN-357 and the other compounds represented by Formula III inhibit glycogen phosphorylase which removes single glucose residues from α-(1,4)-linkages within glycogen molecules (Repasi, J. and A. Szabo, Pyrrolopyridine-2-carboxylic acid amide derivative useful as inhibitor of glycogen phosphorylase, W.I.P. Organization, Editor. 2006, Prosidion Limited. p. 1-32). The product of this reaction is glucose-1-phosphate. Inhibition of glycogen phosphorylase also shifts metabolism towards glycogen storage, thereby decreasing ectopic firing associated with neuropathic pain.

Alternatively, glutamate uptake by astrocytes is driven by a Na+ electrochemical gradient maintained by the Na+/K+ ATPase, and is critically dependent on energy. Intracellular Na+ accumulation activates the Na+/K+ ATPase resulting in an increase in the ADP/ATP ratio and activating glycolysis (Mayo Clinic Proc. 80:1338). At nociceptive synapses, decreased glycolysis would result in poor uptake of glutamate and thereby potentiation of excitatory neurotransmission leading to a chronic pain state. CS-917 and the other compounds represented by Formula I inhibit fructose biphosphatase, an irreversible enzyme in gluconeogenesis that converts fructose-1,6-biphosphate to fructose-6-phosphate (Van Poelje, P.D., M.D. Erion, and T. Fujiwara, A combination of FBPase inhibitors and antidiabetic agents useful for the treatment of diabetes, in World Intellectual Property Organization. 2002, Metabasis Therapeutics Inc. p. 1-392). Since the net effect of fructose biphosphatase inhibition is to increase glycolysis, restored glutamate uptake at nociceptive synapses would relieve neuropathic pain.

### Experimental Results

Three models in rats have been shown to correlate well to clinical outcome both with respect to the rank order of active (Gabapentin, Pregabalin, Amitriptyline, Carbamazepine and N-type Ca++ blockers) and inactive (SSRI and NSAIDs) substances, and also between experimental and effective therapeutic doses. These models are based on three surgical procedures: (i) the spinal nerve ligation (SNL) [Kim, S. and J. Chung, An experimental model for peripheralneuropathy produced by segmental spinal nerve ligation in the rat. Pain, 1992. 50: p. 355-363.]; (ii) the partial sciatic nerve lesion (Seltzer) [Seltzer, Z., R. Dubner, and Y. Shir, A novel behavioral model of neuropathic pain disorders produced in rats by partial sciatic nerve injury. Pain, 1990. 43: p. 205-218.]; (iii) and the chronic constriction injury [Bennett, G. and Y. Xie, A peripheral mononeuropathy in rat that produces disorders of pain sensation like those seen in man. Pain, 1988. 33: p. 87-107.].

Activity of CS-917 and NP-12 will be demonstrated in the SNL model, with the following protocol:

**Table 1 Experimental protocol. (AM, allodynia measurement by von Frey 1 h. post-drug or vehicle administration; GPN, gabapentin; IP, intraperitoneal.)**

| **Day** | **Procedure** | **Drug** | **Notes** |
|---|---|---|---|
| 0 | AM followed by SNL | None | Estabhsh baseline behavior Perform SNL |
| 14 | AM | Vehicle | Confirm stable pain condition |
| 15 | GPN followed by PWT | GPN 100mg/kg IP | Comparator and positive control |
| 16-20 | On each day, dose candidate drug followed by AM | glucose metabolism modulator: *e.g.* CS-917, and NP-12, at 100 mg/kg PO | Candidate drug effect |
| 21 | GPN followed by AM | GPN 100 mg/kg IP | Internal control to confirm any apparent absence of effect for test compound |

### Effect of PSN-357 on mechanical allodynia induced by spinal nerve ligation in rats

Male Sprague-Dawley rats (Hsd:Sprague-Dawley®™SD®™, Harlan, Indianapolis, Indiana, U.S.A.) weighing 223 ± 2 g on Day14 were housed three per cage. Animals had free access to food and water and were maintained on a 12:12h light/dark schedule for the entire duration of the study. The animal colony was maintained at 21°C and 60% humidity. All experiments were conducted in accordance with the International Association for the Study of Pain guidelines and were approved by the University of Minnesota Animal Care and Use Committee.

The Spinal Nerve Ligation (SNL) model was used to induce chronic neuropathic pain. The animals were anesthetized with isoflurane, the left L6 transverse process was removed, and the L5 and L6 spinal nerves were tightly ligated with 6-0 silk suture. The wound was then closed with internal sutures and external staples.

Baseline, post-injury and post-treatment values for non-noxious mechanical sensitivity were evaluated using 8 Semmes-Weinstein filaments (Stoelting, Wood Dale, IL, USA) with varying stiffness (0.4, 0.7, 1.2, 2.0, 3.6, 5.5, 8.5, and 15 g) according to the up-down method. Animals were placed on a perforated metallic platform and allowed to acclimate to their surroundings for a minimum of 30 minutes before testing. The mean and standard error of the mean (SEM) were determined for each paw in each treatment group. Since this stimulus is normally not considered painful, significant injury-induced increases in responsiveness in this test are interpreted as a measure of mechanical allodynia.

Statistical analyses were conducted using Prism™ 4.01 (GraphPad, San Diego, CA, USA). Mechanical hypersensitivity of the injured paw was determined by comparing pre-SNL to post-SNL values at Day14. Data were analyzed using the Wilcoxon test. Effect of vehicle was tested by comparing post-SNL to post-vehicle values using the Wilcoxon test. Drug effect was analyzed by comparing post-vehicle and post-drug values using the Friedman test followed by a Dunn's post hoc test.

PSN-357 was dissolved in dimethyl sulfoxide (Sigma, cat. D8418, batch 105K00451) and diluted with 0.9% sterile saline (Baxter, cat. 2F7124, lot G046730) to the final concentration containing less than 2% dimethyl sulfoxide and ultrasound dispersed for five minutes. PSN-357 and vehicle were administered with a volume of 5 ml/kg.

PSN-357 30 mg/kg PO significantly (p < 0.01 vs. vehicle, Dunn's *post hoc* test) reduced mechanical allodynia on post-SNL day 19.

The dosage of a specific active compound of the invention depends upon many factors that are well known to those skilled in the art, for example, the particular compound; the condition being treated; the age, weight, and clinical condition of the recipient patient; and the experience and judgment of the clinician or practitioner administering the therapy. An effective amount of the compound is that which provides either subjective relief of symptoms or an objectively identifiable improvement as noted by the clinician or other qualified observer. The dosing range varies with the compound used, the route of administration and the potency of the particular compound. For example, for PSN-357, CS-917 and NP-12, the dosing ranges based on pre-clinical and clinical data described (above) would be 3-100 mg/kg, 2-100 mg/kg and 10-100 mg/kg, respectively, administered PO.

### Definitions

The phrase "a" or "an" entity as used herein refers to one or more of that entity; for example, a compound refers to one or more compounds or at least one compound. As such, the terms "a" or (or "an"), "one or more", and "at least one" can be used interchangeably herein.

The terms "optional" or "optionally" as used herein means that a subsequently described event or circumstance may but need not occur, and that the description includes instances where the event or circumstance occurs and instances in which it does not. For example, "optional bond" means that the bond may or may not be present, and that the description includes single, double, or triple bonds.

The term "independently" is used herein to indicate that a variable is applied in any one instance without regard to the presence or absence of a variable having that same or a different definition within the same compound. Thus, in a compound in which R appears twice and is defined as "independently carbon or nitrogen", both R's can be Carbon, both R's can be nitrogen, or one R can be carbon and the other nitrogen.

The term "alkenyl" refers to an unsubstituted hydrocarbon chain radical having from 2 to 10 carbon atoms having one or two olefinic double bonds, preferably one olefinic double bond. The term "C_{2-N} alkenyl" refers to an alkenyl comprising 2 to N carbon atoms where N is an integer having the following values: 3, 4, 5, 6, 7, 8, 9, or 10. The term "C₂₋₁₀ alkenyl" refers to an alkenyl comprising 2 to 10 carbon atoms. Examples include, but are not limited to vinyl, 1-propenyl, 2-propenyl, (allyl) or 2-butenyl (crotyl).

The term alkyl refers to an unbranched or branched chain, saturated, monovalent hydrocarbon residue containing 1 to 30 carbon atoms. The term "C_{1-N} alkyl" refers to an alkyl comprising 1 to N carbon atoms, where N is an integer having the following values: 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30. The term "C₁₋₄" alkyl refers to an alkyl contain 1 to 4 carbon atoms. The term "low alkyl" or "lower alkyl" denotes a straight or branched chain hydrocarbon residue comprising 1 to 8 carbon atoms. "C₁₋₂₀ alkyl" as used herein refers to an alkyl comprising 1 to 20 carbon atoms. "C₁₋₁₀ alkyl" as used herein refers to an alkyl comprising 1 to 10 carbon atoms. Examples of alkyl groups include, but are not limited to, methyl, ethyl, propyl, i-propyl, n-butyl, i-butyl, t-butyl, pentyl, isopentyl, neopentyl, hexyl, heptyl, and octyl. The term (ar)alkyl or (heteroaryl)alkyl indicate the alkyl group is optionally substituted by an aryl or a heteroaryl group respectively.

The term "halogenated alkyl" (or "haloalkyl") refers to an unbranched or branched chain alkyl comprising at least one of F, Cl, Br, and I. The term "C₁₋₃ haloalkyl" refers to a haloalkyl comprising 1 to 3 carbons and at least one of F, Cl, Br, and I. The term "halogenated lower alkyl" refers to a haloalkyl comprising 1 to 8 carbon atoms and at least one of F, Cl, Br, and I. Examples include, but are not limited to, fluoromethyl, chloromethyl, bromomethyl, iodomethyl, difluoromethyl, dichloromethyl, dibromomethyl, diiodomethyl, trifluoromethyl, trichloromethyl, tribromomethyl, triiodomethyl, 1-fluoroethyl, 1-chloroethyl, 1-bromoethyl, 1-iodoethyl, 2-fluoroethyl, 2-chhoroethyl, 2-bromoethyl, 2-iodoethyl, 2,2-difluoroethyl, 2,2-dichloroethyl, 2,2-dibromoethyl, 2,2-diiodoethyl, 3-fluoropropyl, 3-chloropropyl, 3-bromopropyl, 3-iodopropyl, 2,2,2-trifluoroethyl, 1,1,2,2,2-pentafluoroethyl, 1-fluoro-1-chloroethyl, or 1-fluroro-1-chloro-1-bromoethyl.

The term "alkynyl" refers to an unbranched or branched hydrocarbon chain radical having from 2 to 10 carbon atoms, preferably 2 to 5 carbon atoms, and having one triple bond. The term "C_{2-N} alkynyl" refers to an alkynyl comprising 2 to N carbon atoms, where N is an integer having the following values: 2, 3, 4, 5, 6, 7, 8, 9, or 10. The term "C₂₋₄ alkynyl" refers to an alkynyl comprising 2 to 4 carbon atoms. The term "C₂₋₁₀ alkynyl" refers to an alkynyl comprising 2 to 10 carbon atoms. Examples include, but are not limited to, ethynyl, 1-propynyl, 2-propynyl, 1-butynyl, 2-butynyl, or 3-butynyl.

The term "cycloalkyl" refers to a saturated carbocyclic ring comprising 3 to 8 carbon atoms, i.e. cyclopropyl, cyclobutyl, cyclopentyl, cyclohexyl, cycloheptyl or cyclooctyl. The term "C₃₋₇ cycloalkyl" as used herein refers to a cycloalkyl comprising 3 to 7 carbons in the carbocyclic ring.

The term "alkoxy" refers to an -O-alkyl group, wherein alkyl is defined above. Examples include, but are not limited to, methoxy, ethoxy, n-propyloxy, i-propyloxy, n-butyloxy, i-butyloxy, t-butyloxy. "Lower alkoxy" or "low alkoxy" or "low alkoxyl" as used herein denotes an alkoxy group with a "lower alkyl" group as previously defined. "C₁₋₁₀ alkoxy" refers to an -O-alkyl wherein alkyl is C₁₋₁₀.

The term "substituted", as used herein, means that one or more hydrogens on the designated atom is replaced with a selection from the indicated group, provided that the designated atom's normal valency is not exceeded, and that the substitution results in a stable compound.

The term "halo" or as used herein includes fluoro, chloro, bromo, and iodo.

The term "pharmaceutically acceptable salt or prodrug" is used throughout the specification to describe any pharmaceutically acceptable form (such as an ester, phosphate ester, salt of an ester or related group) of a compound which upon administration to a mammal, provides the active compound. Pharmaceutically acceptable salts include those derived from pharmaceutically acceptable inorganic or organic bases and acids. Pharmaceutically acceptable prodrugs refer to a compound that is metabolized, for example hydrolyzed or oxidized, in the host to form a compound of a method of the present invention. A "pharmaceutically acceptable salt" form of an active ingredient may also initially confer a desirable pharmacokinetic property on the active ingredient which was absent in the non-salt form, and may even positively affect the pharmacodynamics of the active ingredient with respect to its therapeutic activity in the body. The phrase "pharmaceutically acceptable salt" of a compound as used herein means a salt that is pharmaceutically acceptable and that possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as glycolic acid, pyruvic acid, lactic acid, malonic acid, maleic acid, fumaric acid, tartaric acid, citric acid, 3-(4-hydroxybenzoyl)benzoic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, lauryl sulfuric acid, gluconic acid, glutamic acid, salicyclic acid, muconic acid, and the like or (2) basic addition salts formed with the conjugate bases of any of the inorganic acids listed above, wherein the conjugate bases comprise a cationic component selected from among Na⁺, K⁺, Mg2⁺, Ca2⁺, NHgR"'4-g⁺, in which R'" is a C₁₋₃ alkyl and g is a number selected from among 0, 1, 2, 3, or 4. It should be understood that all references to pharmaceutically acceptable salts include solvent addition forms (solvates), water addition forms (hydrates), or crystal forms (polymorphs) as defined herein, of the same acid additions salts.

Any of the compounds described herein can be administered as a prodrug to increase the activity, bioavailability, stability or otherwise alter the properties of the selected compound. A number of prodrug ligands are known.

The compounds used in methods of the present invention may be formulated in a wide variety of oral administration dosage forms and carriers. Oral administration can be in the form of tablets, coated tablets, hard and soft gelatin capsules, solutions, emulsions, syrups, or suspensions. Compounds used in methods of the present invention are efficacious when administered by suppository administration, among other routes of administration. The most convenient manner of administration is generally oral using a convenient daily dosing regimen which can be adjusted according to the severity of the pain.

A compound or compounds used in methods of the present invention, as well as their pharmaceutically acceptable salts, solvates, hydrates, prodrugs, and polymorphs, together with one or more conventional excipients, carriers, or diluents, may be placed into the form of pharmaceutical compositions and unit dosages. The pharmaceutical compositions and unit dosage forms may be comprised of conventional ingredients in conventional proportions, with or without additional active compounds and the unit dosage forms may contain any suitable effective amount of the active ingredient commensurate with the intended daily dosage range to be employed. The pharmaceutical compositions may be employed as solids, such as tablets or filled capsules, semisolids, powders, sustained release formulations or liquids such as suspensions, emulsions, or filled capsules for oral use; or in the form of suppositories for rectal or vaginal administration. A typical preparation will contain from about 5% to about 95% active compound or compounds (w/w). The term "preparation or "dosage form" is intended to include both solid and liquid formulations of the active compound and one skilled in the art will appreciate that an active ingredient can exist in different preparations depending on the desired dose and pharmacokinetic parameters.

The term "excipient" as used herein refers to a compound that is used to prepare a pharmaceutical composition, and is generally safe, non-toxic and neither biologically nor otherwise undesirable, and includes excipients that are acceptable for veterinary use as well as human pharmaceutical use. The compounds of this invention can be administered alone but will generally be administered in admixture with one or more suitable pharmaceutical excipients, diluents or carriers selected with regard to the intended route of administration and standard pharmaceutical practice.

Solid form preparations include powders, tablets, pills capsules, suppositories, and dispersible granules. A solid carrier may be one or more substances which may also act as diluents, flavoring agents, solubilizers, lubricants, suspending agents, binders, preservatives, tablet disintegrating agents, or an encapsulating material. In powders, the carrier generally is a finely divided solid which is a mixture with the finely divided active component. In tablets, the active component generally is mixed with the carrier having the necessary binding capacity in suitable proportions and compacted in the shape and size desired. Suitable carriers include but are not limited to magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. Solid form preparations may contain, in addition to the active component colorants, flavors, stabilizers, buffers, artificial and natural sweeteners, dispersants, thickeners, solubilizing agents, and the like.

Liquid formulations also are suitable for oral administration include liquid formulations including emulsions, syrups, elixirs and aqueous suspensions. These include solid form preparations which are intended to be converted to liquid form preparations shortly before use. Emulsions may be prepared in solutions, for example, in aqueous propylene glycol solutions or may contain emulsifying agents such as lecithin, sorbitan monooleate, or acacia. Aqueous suspensions can be prepared by dispersing the finely divided active component in water with viscous material, such as natural or synthetic gums, resins, methylcellulose, sodium carboxymethylcellulose, and other well known suspending agents.

The compounds used in methods of the present invention may be formulated for administration as suppositories. A low melting wax, such as a mixture of fatty acid glycerides or cocoa butter is first melted and the active component is dispersed homogeneously, for example, by stirring. The molten homogeneous mixture is then poured into convenient sized molds, allowed to cool and to solidify.

The compounds used in methods of the present invention may be formulated for vaginal administration. Pessaries, tampons, creams, gels, pastes, foams or sprays containing in addition to the active ingredient such carriers as are known in the art to be appropriate.

Suitable formulations along with pharmaceutical carriers, diluents and excipients are described in Remington: The Science and Practice of Pharmacy 1995, edited by E. W. Martin, Mack Publishing Company, 19th Edition, Easton, Pennsylvania, which is hereby incorporated by reference. A skilled formulation scientist may modify the formulations within the teachings of the specification to provide numerous formulations for a particular route of administration without rendering the compositions of the present invention unstable or comprising their therapeutic activity.

The modification of the present compounds to render them more soluble in water or other vehicle, for example, may be easily accomplished by minor modifications (e.g., salt formulation), which are well within the ordinary skill in the art. It is also well within the ordinary skill of the art to modify the route of administration and dosage regimen of a particular compound in order to manage the pharmacokinetics of the present compounds for maximum beneficial effect in patients.

The term "medicament" means a substance used in a method of treatment and/or prophylaxis of a subject in need thereof, wherein the substance includes, but is not limited to, a composition, a formulation, a dosage from, and the like, comprising a compound of formulas I, II or III. It is contemplated that the use of a compound of a method of the invention in the manufacture of a medicament for the treatment of any of the conditions disclosed herein can be any of the compounds contemplated in any of the aspects of the invention, either alone or in combination with other compounds of the methods of the present invention.

The term "therapeutically effective amount" as used herein means an amount required to reduce symptoms of pain, particularly neuropathic pain, in an individual. The dose will be adjusted to the individual requirements in each particular case. That dosage can vary within wide limits depending upon numerous factors such as the severity of the condition to be treated, the age and general health condition of the patient, other medicaments with which the patient is being treated, the route and form of administration and the preferences and experience of the medical practitioner involved. For oral administration, a daily dosage of between about 0.1 and about 10g, including all values in between, such as 0.25, 0.5, 0.75, 1, 1.5, 2, 2.5, 3, 3.5, 4, 4.5, 5, 5.5, 6, 6.5, 7, 7.5, 8, 8.5, 9, and 9.5, per day should be appropriate in monotherapy and/or in combination therapy. A preferred daily dosage is between about 0.5 and about 7.5g per day, a more preferred dosage is between 1.5 and about 6.0g per day. One of ordinary skill in treating conditions described herein will be able, without undue experimentation and in reliance on personal knowledge, experience, and the disclosures of this application, to ascertain a therapeutically effective amount of the compounds of the methods of the present invention for a given condition and patient.

## Claims

1. A glycogen phosphorylase-inhibiting compound of structural formula III: 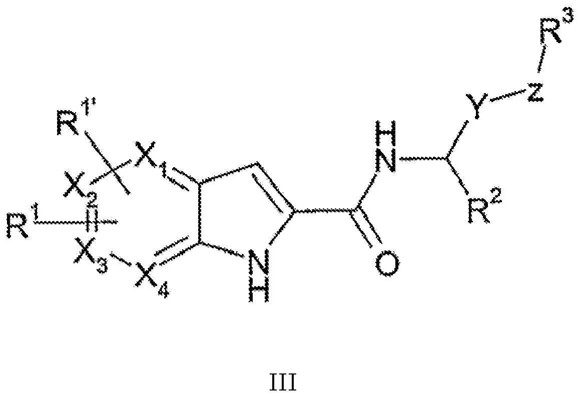 wherein:
one of X1, X₂, X₃ and X₄ must be N and the others must be C;
R¹ and R^{1'} are each independently, halogen, hydroxy, cyano, C₀₋₄alkyl, C₁₋₄alkoxy, fluoromethyl, difluoromethyl, trifluoromethyl, ethenyl, or ethynyl;
R² is C0-4alkyl, COOR6, COR6, C₁₋₄alkoxyC₁₋₄alkyl-, hydroxyC₁₋₄alkyl-, cycloalkylC₀₋₄alkyl-, arylC₀₋₄alkyl-, hetarylC₀₋₄alkyl-, wherein any of the aryl or
hetaryl rings are optionally substituted with 1-2 independent halogen, cyano, C₀₋₄alkyl, C₁₋₄alkoxy, -N(C₀₋₄alkyl)(C₀₋₄alkyl),-SO₂C₁₋₄alkyl,-SO₂N(C₀₋₄alkyl)(C₀₋₄alkyl), hydroxy, fluoromethyl, difluoromethyl, or trifluoromethyl substituents;
Y is C₀₋₂alkyl or-CH(OH)- ;
Z is CH₂, -C(O)-, -O-, >N(C₀₋₄alkyl), >N(C₃₋₆cycloalkyl), or absent; but when Y is -CH(OH) -, Z or R³ must be bonded to Y through a carbon-carbon bond;
R³ is hydrogen, -COOC₀₋₄alkyl, C₁₋₄alkoxy, C₁₋₄alkyl, arylC₁₋₄alkylthio-, -C₀₋₄alkylaryl, -C₀₋₄alkylhetaryl, -C₀₋₄alkylcycloalkyl or -C₀₋₄alkylheterocyclyl, wherein any of the rings is optionally substituted with 1-3 independent halogen, cyano, C₁₋₄alkyl, fluoromethyl, difluoromethyl, trifluoromethyl, -C₀₋₄alkylNHC(O)O(C₁₋₄alkyl), -C₀₋₄alkylNR⁷R⁸, -C(O)R⁹, C₁₋₄alkoxyC₀₋₄alkyl-, - COOC₀₋₄alkyl, -C₀₋₄alkylNHC(O)R⁹, -C₀₋₄alkylC(O)N(R¹⁰)₂, -C₁₋₄alkoxyC₁₋₄alkoxy, hydroxyC₀₋₄alkyl-, -NHSO₂R¹⁰, -SO₂(C₁₋₄alkyl), -SO₂NR¹¹R¹², 5- to 6-membered heterocyclyl, phenylC₀₋₂alkoxy, or phenylC₀₋₂alkyl substituents,
wherein phenyl is optionally substituted with 1-2 independent halogen, cyano, C₁₋₄alkyl, C₁₋₄alkoxy, -N(C₀₋₄alkyl)(C₀₋₄alkyl), -S0₂C₁₋₄alkyl, -S0₂N(C₀₋₄alkyl)(C₀₋₄alkyl), hydroxy, fluoromethyl, difluoromethyl or trifluoromethyl substituents,
or two bonds on a ring carbon of the heterocyclyl group optionally can form an oxo (=O) substituent;
or R³ is-NR⁴(-C₀₋₄alkylR⁵) ;
R⁴ is C₀₋₃alkyl, -C₂₋₃alkyl-NR⁷R⁸, C₃₋₆cycloalkyl optionally substituted by hydroxyC₀₋₄alkyl-further optionally substituted by hydroxy, C₁₋₂alkoxyC₂₋₄alkyl-, or C₁₋₂alkyl-S(O)ₙ-C₂₋₃alkyl-;
n is 0, 1, or 2 ;
R⁵ is hydrogen, hydroxyC₂₋₃alkyl-, C₁₋₂alkoxyC₀₋₄alkyl-, or aryl, hetaryl, or heterocyclyl;
wherein a heterocyclic nitrogen-containing R⁵ ring optionally is mono-substituted on the ring nitrogen with C₁₋₄alkyl, benzyl, benzoyl, C₁₋₄alkyl-C(O)-, -SO₂C₁₋₄alkyl, -SO₂N(C₀₋₄alkyl)(C₀₋₄alkyl), C₁₋₄alkoxycarbonyl or aryl(C₁₋₄alkoxy)carbonyl; and wherein the R⁵ rings are optionally mono-substituted on a ring carbon with halogen, cyano, C₁₋₄alkyl-C(O) -, C₁₋₄alkyl-SO₂-, C₁₋₄alkyl, C₁₋₄alkoxy, hydroxy, -N(C₀₋₄alkyl)(C₀₋₄alkyl), hydroxyC₀₋₄alkyl-, or C₀₋₄alkylcarbamoyl-, provided that no quaternised nitrogen is included; or two bonds on a ring carbon of the heterocyclyl group optionally can form an oxo (=O) substituent;
R⁶ is C₁₋₄alkyl, aryl, or hetaryl;
R⁷ and R⁸ are independently C₀₋₄alkyl, C₃₋₆cycloalkyl, or CO(C₁₋₄alkyl) ;
R⁹ is C₁₋₄alkyl or C₃₋₆cycloalkyl ;
R¹⁰ is C₀₋₄alkyl or C₃₋₆cycloalkyl ;
R¹ and R¹² are independently C₀₋₄alkyl or together with the nitrogen to which they are attached may form a 4-to 6-membered heterocycle; and
wherein there are no nitrogen-oxygen, nitrogen-nitrogen or nitrogen-halogen bonds in linking the three components -Y-Z-R³ to each other;
or a pharmaceutically acceptable salt, solvate, ester or hydrate thereof, for use in a method of treating pain.

2. A compound for use as claimed in claim 1, wherein the compound is 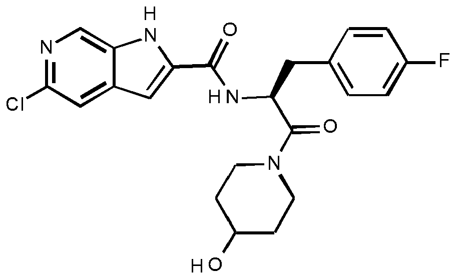 or a pharmaceutically acceptable salt, solvate or hydrate thereof.

3. A compound for use as claimed in claim 1 or 2, wherein the mammal is a human.

4. A compound for use as claimed in any one of the preceding claims, wherein the pain is neuropathic pain.

5. A compound for use as claimed in any one of the preceding claims, wherein the treatment of pain comprises administering a pharmaceutical composition comprising the compound to a mammal in need of such treatment.

6. A compound for use as claimed in claim 5, the composition further comprises at least one pharmaceutically acceptable additive selected from the group consisting of adjuvant, excipient, diluent, and carrier.
